# EUROPÄISCHE PATENTSCHRIFT

(11) **EP 1 804 965 B1**
(45) Veröffentlichungstag und Bekanntmachung des Hinweises auf die Patenterteilung: **09.09.2009**
(21) Anmeldenummer: 05797972.6
(22) Anmeldetag: 21.10.2005
(51) Int. Cl.: B01J 20/10, B01D 53/04, B01D 53/12

(54) **VERWENDUNG VON HYDROPHOBIERTEN KIESELGEL ALS SELEKTIVES SORBENS ZUR ENTFERNUNG ORGANISCHER SILIZIUMVERBINDUNGEN**
USE OF HYDROPHOBIZED SILICA GEL AS A SELECTIVE SORBENT FOR REMOVING ORGANIC SILICON COMPOUNDS
UTILISATION DE GEL DE SILICE HYDROPHOBE COMME SORBANT SELECTIF POUR ELIMINER DES COMPOSÉS DE SILICIUM ORGANIQUES

(30) Priorität: 25.10.2004 DE 102004051807
(43) Veröffentlichungstag der Anmeldung: 11.07.2007
(73) Patentinhaber: Fraunhofer-Gesellschaft zur Förderung der angewandten Forschung e.V., 80686 München (DE)
(72) Erfinder: URBAN, Wolfgang, 45894 Gelsenkirchen (DE); UNGER, Christoph, 46485 Wesel (DE)
(74) Vertreter: Pfenning, Meinig & Partner GbR
(86) Internationale Anmeldenummer: PCT/EP2005/011361
(87) Internationale Veröffentlichungsnummer: WO 2006/045561

(56) Entgegenhaltungen:
- EP-A- 0 903 171
- EP-A- 1 316 350
- DE-A1- 10 224 349
- SCHWEIGKOFLER M.: "Removal of siloxanes in biogases" JOURNAL OF HAZARDOUS MATERIALS, Bd. 83, Nr. 3, 30. Mai 2001 (2001-05-30), Seiten 183-196, XP002362422 in der Anmeldung erwähnt
- PATENT ABSTRACTS OF JAPAN Bd. 2002, Nr. 06, 4. Juni 2002 (2002-06-04) & JP 2002 060767 A (NKK CORP), 26. Februar 2002 (2002-02-26)
- PATENT ABSTRACTS OF JAPAN Bd. 010, Nr. 090 (C-337), 8. April 1986 (1986-04-08) & JP 60 222144 A (MATSUSHITA DENKO KK), 6. November 1985 (1985-11-06)

## Beschreibung

Die Erfindung betrifft die Verwendung von hydrophobiertem Kieselgel als selektivem Sorbens für siliziumorganische Verbindungen in anthropogenen oder biogenen, methanhaltigen Gasen. Hierzu zählen u.a. Biogas, Klärgas oder Deponiegas.

Bei der Nutzung biogener oder anthropogener methanhaltiger Gase, wie Biogas, Klärgas oder Deponiegas, sind zahlreiche durch enthaltene Minorkomponenten verursachte technische Probleme zu lösen. Besonders hervorzuheben sind hier siliziumorganische Verunreinigungen, die mit steigender Tendenz in allen vorgenannten Gasen auftreten und in besonderem Maße die energetische Nutzung der methanhaltigen Gase - z.B. durch sandartige Ablagerungen in Motoren - erschweren. In Klärwerken und Deponien mit hoher Belastung mit Siliziumorganika (Siloxane) mussten deshalb in der Vergangenheit hohe Wartungskosten von Motoren toleriert oder sogar ganz auf die Verwertung des Methans verzichtet werden. Daher ist eine vorhergehende Aufbereitung und Reinigung biogener Gase vor der energetischen Nutzung in vielen Fällen unvermeidbar.

Stand der Technik sind adsorptive Verfahren mit dem Sorptionsmittel Aktivkohle, oft auch in Kombination mit einer vorgelagerten Gaskühlung oder Gaswäsche. Mit diesen Verfahren werden die technischen Anforderungen an die Gasqualität erfüllt. Allerdings erreichen diese Gasreinigungsverfahren nicht die wirtschaftlichen Anforderungen.

Kieselgele weisen wesentlich höhere Beladungskapazitäten hinsichtlich Siliziumverbindungen auf, haben jedoch den entscheidenden Nachteil, dass sie ausgeprägt hydrophil sind. Eine Abtrennung der Siliziumverbindungen aus biogenen Gasen mit einer parallelen Gastrocknung ist jedoch unwirtschaftlich. Die Erfindung beschreibt daher die Anwendung hydrophobierten Kieselgels zur Abtrennung von organischen Siliziumverbindungen aus methanhaltigen Gasen. Desweiteren wird auch ein Verfahren zur Regenerierung des verbesserten und effizienteren Sorptionsmittels beschrieben.

Stand der Technik im Bereich Reinigung methanhaltiger Gase, z.B. die Aufbereitung für eine energetische Nutzung in Motoren, Turbinen oder Brennstoffzellen, sind adsorptive Verfahren mit Aktivkohlen und absorptive Verfahren, z.B. Gaswäsche mit Sickerwasser, alkalischem Wasser oder modifizierten Heizölen bzw. organischen Lösungsmitteln. Adsorptive Verfahren werden oftmals mit einer vorgelagerten Gaskühlung oder absorptive Verfahrensschritten gekoppelt. Die bisher verwendeten Gasreinigungsverfahren waren hinsichtlich Wirtschaftlichkeit oder Verfügbarkeit nicht erfolgreich. So sind im wesentlichen drei Probleme nicht gelöst worden:
- zu hohe Betriebskosten durch zu hohen Verbrauch an Adsorbentien (Aktivkohle),
- nicht erreichte Reinigungsleistungen bei absorptiven Wäscheverfahren oder
- zu geringe Betriebssicherheit durch hohe Störanfälligkeiten (Gastiefkühlung).

Aktivkohlen sind zur Entfernung der Siliziumorganika aus biogenen bzw. methanhaltigen Gasen aufgrund ihrer Unselektivität, konkurrierender Adsorption (Vielstoffgemisch) und - sich daraus ergebend - ihrer sehr geringen Adsorptionskapazität bezüglich Siloxanen (ca. 1 Gew.%) nur bedingt geeignet. Sie erfüllen zwar die technischen Anforderungen, jedoch nicht die wirtschaftlichen Erwartungen.

Absorptive Verfahren verursachen einen hohen apparatetechnischen Aufwand und sind erst bei größeren Volumenströmen anzuwenden. Siloxane sind chemisch äußerst beständig und können nur mit starken Säuren oder Basen angegriffen werden. Aufgrund des hohen CO₂-Anteils in biogenen methanhaltigen Gasen kommen nur hochkonzentrierte Säuren in Betracht. Entsprechende Vorversuche mit einer maximalen Abscheideeffizienz bis 95% hinsichtlich ausgewählter organischer Siliziumverbindungen (das Siloxan L2, Hexamethyldisiloxan und das D5, Decamethylcyclopentasiloxan) wurden bereits untersucht [Schweigkofler, M.: Bestimmung flüchtiger Siliziumverbindungen in Biogasen mittels Kanisterprobennahme und GC-MSD/AED-Analytik. TU München, Dissertation, 2000]. Geeignet wären prinzipiell hochkonzentrierte Schwefelsäure, Trichloressigsäure oder Salpetersäure. Allerdings erzeugt der Einsatz von hochkonzentrierten Säuren als Waschmittel (in Füllkörperkolonnen, die überdies bei höheren Temperaturen betrieben werden müssten) Korrosionsprobleme, da nicht ausgeschlossen werden kann, dass diese in nachfolgende Bauteile vordringen. Gleiches gilt für den Einsatz von Heizöl oder prinzipiell geeigneten organischen Lösungsmitteln. Die Betriebserfahrungen waren sehr zwiespältig: die Abscheideleistungen sind mäßig bis befriedigend und der Austrag an Waschmittel in nachfolgende Aggregate erheblich. Darüber hinaus muss festgestellt werden, dass mit absorptiven Deponiegasreinigungsverfahren allein die Anforderungen an die Gasqualität nicht erreicht werden können. Auch bei diesen Verfahren sind Aktivkohlen nachzuschalten.

Deshalb wurden in der jüngeren Vergangenheit intensive Anstrengungen unternommen, die Abtrennungsleistung einer adsorptiven Gasreinigung durch zusätzliche Verfahrensschritte zu verbessern. Fraunhofer UMSICHT hat hier beispielsweise in Zusammenarbeit mit der Siloxa AG eine Gasreinigungsanlage (Basis Aktivkohle) in Kombination mit einer Gastiefkühlung (Kühlung des Gases auf -30 °C, Projekt Köln-Rodenkirchen) bzw. mit einer Sickerwasserwäsche entwickelt und getestet. Durch die Erweiterung der Gasreinigungsanlage konnte die wirtschaftliche Effizienz spürbar verbessert werden; jedoch zum Preis erhöhter Kapitalkosten, da ein zusätzlicher Verfahrensschritt notwendig wird.

Mit einer vorgelagerten Gaskühlung bzw. mit einer vorgelagerten Sickerwasserwäsche können die wasserlöslichen Silanole quantitativ entfernt werden. Die Silanole machen jedoch höchstens 50% der in Deponiegasen befindlichen Siliziumorganika aus. Siloxane können mit den vorgenannten Verfahrensstufen nicht entfernt werden. Desweiteren muss darauf hingewiesen werden, dass in Klärgasen keine Silanole (Abbauprodukte der Siloxane) zu finden sind und diese Verfahrensstufen nicht für eine effiziente Klärgasaufbereitung geeignet sind. Schließlich werden mit Tieftemperatur- bzw. absorptiven Verfahren keine Aromaten, die hauptsächlich einer effizienten Abtrennung von Siliziumorganika aus Deponie- und Klärgasen mit Aktivkohle entgegenstehen, aus dem Gas entfernt.

Gegenwärtig wird daran gearbeitet, katalytisch wirkende Feststoffe in Füllkörperkolonnen (Sickerwasserwäsche) auf Ihre Eignung zum beschleunigten hydrolytischen Abbau von Siloxanen zu Silanolen, also wasserlöslichen siliziumorganischen Bestandteilen, zu testen. Ergebnisse aus diesen Untersuchungen liegen noch nicht vor.

Die Effizienz verfügbarer Gasreinigungsanlagen genügt somit bisher nur teilweise den Anforderungen und sollte wesentlich verbessert werden, um eine weitere Deponiegas- bzw. Klärgasnutzung zu ermöglichen.

Ein erfolgsversprechender Lösungsansatz für ein effizienteres Sorbensmaterial ergibt sich aus Untersuchungen, bei denen verschiedene Aktivkohlen, Molekularsiebe und Kieselgel als Adsorbens zur selektiven Siloxanentfernung aus Biogasen untersucht wurden (Schweigkofler, M.; Niessner, R.: Removal of siloxanes in biogases. Journal of Hazardous Materials B83 (2001) 183-196). Es konnte nachgewiesen werden, dass Kieselgele als einzige Adsorbentien Siliziumorganika quantitativ adsorbieren und darüber hinaus eine mindestens 10-fach höhere Beladungskapazität als Aktivkohlen ermöglichen. Allerdings ist die Beladungskapazität stark von der Feuchte im Gas abhängig - mit zunehmender Gasfeuchte nimmt die Beladungskapazität hinsichtlich Siloxanen rapide ab - weshalb Kieselgeladsorber als alleinige Prozessstufe nicht geeignet sind.

Die Wasserentfernung mittels vorgeschalteter Gastrockung führt dabei auf den von der Aktivkohle bekannten und problembehafteten Weg.

Silanisierte Kieselgele sind Stand der Technik im Bereich der instrumentellen Analytik, z.B. in der Gaschromatographie. Allerdings sind auf dem Markt verfügbare Qualitäten von silanisierten Kieselgelen (Korngrößen < 50 µm, höchste Reinheiten > 99,95 %, Preise > 250 €/kg) für die angestrebte Anwendung kaum geeignet. Benötigt werden Korngrößen von 2 bis 5 mm für Fest- und Wanderbettreaktoren oder 0,2 bis 0,8 mm bei Auslegung als Wirbelbettreaktor (geforderter geringer Druckverlust über der Adsorbensschüttung) und wesentlich geringere Anschaffungskosten (möglichst vergleichbar denen von Aktivkohlen, < 10-20 €/kg).

Die silanisierende Hydrophobierung von Kieselgelen außerhalb chromatographischer Anwendungen, beschränkt sich auf funktionalisierte Silane als Ankergruppen zwischen Katalysatoren und Gelen, vorwiegend im Bereich medizinischer Wirkstoffe. Weitere Silanisierungen im Umfeld solcher Katalysezentren werden hydrophobiert, um entweder den Transport des Reaktanden zum Katalysator zu verbessern oder Reaktionen mit den Silanolgruppen zu verhindern. Zur Silanisierung anderer Silikate, wie Zeolithen und Schichtsilikaten existiert Literatur, die sich mit der Selektivitätssteuerung der Katalyse an den Aciden oder Zentren beschäftigt.

Als weiteres wichtiges Einsatzfeld der Silanisierung ist die Haftverbesserung von keramischen Füllstoffen in organischen Matrices zu nennen, die technische Bedeutung, z.B. in der Dentalmedizin, aber auch bei der Produktion von rollwiderstandsarmen Reifen haben.

Auch die Hydrophobierung von Gläsern ist umfangreich beschrieben. In diesen Fällen spielt aber der Erhalt von Poren bzw. ihre Zugänglichkeit keine Rolle.

Ausgehend hiervon war es Aufgabe der vorliegenden Erfindung, die Entfernung organischer Siliziumverbindungen zu erreichen, die die genannten, aus dem Stand der Technik bekannten Nachteile von bisher üblichen Sorptionseinheiten aus Aktivkohle beseitigt und mit geringeren Kosten für Reinigungsverfahren biogener und anthropogener Gase verbunden ist.

Diese Aufgabe wird durch die Verwendung von hydrophobiertem Kieselgel als selektivem Sorbens für siliziumorganische Verbindungen gemäß Anspruch 1 gelöst. Die weiteren abhängigen Ansprüche zeigen vorteilhafte Weiterbildungen auf.

Erfindungsgemäß wird zur Entfernung organischer Siliziumverbindungen aus biogenen und/oder anthropogenen Gasen ein Sorbens aus hydrophobiertem Kieselgel mit einem mittleren Porenradius von 1 bis 40 nm in einer entsprechenden Vorrichtung für die Sorption verwendet.

Mit der Erfindung steht ein deutlich verbessertes Sorbensmaterial zur Aufbereitung von biogenen bzw. anthropogenen methanhaltigen Gasen zur Verfügung.

Das bislang nach dem Stand der Technik verwendete Kieselgel ist bedingt durch seine Hydrophilität für die vorbeschriebenen Einsatzzwecke technisch nicht einsetzbar. Durch die Silanisierung wird das Kieselgel kostengünstig hydrophobiert und damit als Sorbensmaterial zur Siloxanadsorption tauglich gemacht.

Mit der Erfindung wird eine deutliche Reduzierung der Herstellungskosten für hydrophobiertes Kieselgel erreicht und damit ein wirtschaftlicher Einsatz als Sorbensmaterial für eine Gasaufbereitung überhaupt erst möglich. Durch die entsprechende Auswahl des Ausgangsmaterials (Kieselgel) mit einer definierten Porenradienverteilung und einer entsprechenden Behandlung mit einer abgestimmten Rezeptur des Silanisierungsgemisches sollen die bisherigen Herstellungskosten für hydrophobes Kieselgel (für chromatographische Anwendungen bisher mehr als 250 €/kg) minimal um den Faktor 10 reduziert werden.

Die Herstellungskosten des entwickelten Sorbensmaterials (hydrophobiertes Kieselgel) erhöhen sich im Vergleich zu Aktivkohlen um den Faktor 2 bis 2,5; ziehen aber keine weiteren Kapitalkosten zur Umrüstung bereits bestehender Gasreinigungsanlagen nach sich, da beispielsweise das Sorbens in den Festbettadsorbern lediglich auszutauschen ist. Bei neu zu errichtenden Gasreinigungsanlagen reduziert sich der Kapitalbedarf im Vergleich zu bisherigen Anlagen, da die Vorreinigungsstufen, d.h. Wäsche oder Gastiefkühlung, entfallen können. Dem gegenüber erhöhen sich die Adsorptionskapazitäten des entwickelten Sorbensmaterials im Vergleich zu Aktivkohlen um den Faktor 10.

Die wirtschaftliche Effizienz bisheriger Gasreinigungsanlagen zur Aufbereitung von biogenen bzw. anthropogenen methanhaltigen Gasen erhöht sich deutlich bei leicht erhöhten spezifische Adsorbenskosten, ähnlichem oder verringertem Investitionsbedarf und wesentlich verringerten Betriebskosten. Die technischen Anforderungen an die Gasqualität werden eingehalten.

Die Betriebskosten können durch eine extraktive Regenerierung des verwendeten hydrophobierten Kieselgels noch weiter reduziert werden. Bisher wurden die verwendeten Aktivkohlen nach Gebrauch entsorgt und verbrannt, da eine Regenerierung technisch zu aufwendig war bzw. sich die Beladungskapazitäten nach erfolgter Regenerierung deutlich verminderten.

Von besonderem Vorteil ist, dass das effizientere Sorbensmaterial diesen ökonomischen Mehrwert mit neuen Maßstäben beim lokalen und globalen Umweltschutz kombiniert. Anthropogene Schadgasemissionen werden durch eine vermehrte Nutzung methanhaltiger biogener Gase (z.B. Deponie-, Bio- und Klärgas) reduziert und der Umweltschutzbeitrag erreicht dank einer einfacheren Regenerierung und Wiederverwendbarkeit des Sorbensmaterials Bestnoten.

Das entwickelte Sorbensmaterial (hydrophobiertes Kieselgel) ist nicht brennbar und bietet damit nicht zu unterschätzende sicherheitstechnische Vorteile. Adsorberbrände können damit sicher ausgeschlossen werden.

Das hydrophobierte Kieselgel adsorbiert selektiv - keine Wasserdampfadsorption wie bei konventionellem Kieselgel bzw. konkurrierende Adsorption von Aromaten oder höheren Kohlenwasserstoffen wie bei Aktivkohlen - siliziumorganische Verbindungen aus biogenen bzw. anthropogenen methanhaltigen Gasen und weist gegenüber dem Stand der Technik deutlich höhere Beladungskapazitäten auf.

Das Sorbens ist im Vergleich zu der aus dem Stand der Technik bekannten Aktivkohle leicht extraktiv regenerierbar und damit mehrfach wiederverwendbar.

Das Sorbensmaterial kann insbesondere in bereits bestehende Gasreinigungsanlagen durch Austausch der Aktivkohle durch hydrophobiertes Kieselgel integriert werden. Damit können eventuelle Kapitalkosten zur Umrüstung bestehender Anlagen gering gehalten werden. Durch die erwartete vielfach höhere Effizienz des hydrophobierten Kieselgels können die Betriebskosten entscheidend reduziert werden. Das Sorbens ermöglicht die energetische Nutzung von sehr stark mit Siliziumverbindungen belasteten - und deshalb bisher nicht genutzten - methanhaltigen Gasen zu reduzierten Kosten.

Für einen Einsatz von hydrophobiertem Kieselgel als Sorbens zur selektiven Entfernung von Siloxanen spricht u.a. die Tatsache, dass cyclische Siloxane, wie das D4 (Octamethylcyclotetrasiloxan) oder das D5 (Decamethylcyclopentasiloxan), kaum abbaubar bzw. hydrolisierbar sind (vergleiche die o.g. katalytische Hydrolyse von Siloxanen mit Sickerwasser oder anderen wässrigen Medien). Darüber hinaus ist der hydrolytisch nukleophile Angriff auf die Si-O Bindung des cyclischen Silocans sterisch sehr stark behindert, weil die Ringform selbst chemisch sehr stabil und zusätzlich vollständig methyliert, also gesättigt und hydrophob ist. Diese Siloxane lösen sich nicht in wässrigen Medien. Im Gegensatz dazu, steigen mit zunehmender Molekülmasse und zunehmender Hydrophobie des Siloxans (z.B. D4 oder D5) die Van-der-Waals'schen Bindungskräfte deutlich an, was wiederum sehr positive Auswirkungen auf die Sorptionswechselwirkungen zwischen cyclischen Siloxanen und hydrophobiertem Kieselgel hat und hydrophobiertes Kieselgel daher als Sorbensmaterial äußerst geeignet erscheinen lässt.

Vorzugsweise besteht das Sorbens aus einem Kieselgel, das eine Korngröße im Bereich von 0,2 bis 5 mm aufweist. Dabei sind für Fest- und Wanderbettreaktoren Korngrößen im Bereich von 2 bis 5 mm, für Wirbelbettreaktoren Korngrößen im Bereich von 0,2 bis 0,8 mm bevorzugt. Besonders bevorzugt weist das silanisierte Kieselgel einen mittleren Porenradius von 2 bis 8 nm auf.

Das Kieselgel ist bevorzugt mit Silanverbindungen der allgemeinen Formel I

RₙS iXₘH₄₋ₙ₋ₘ I

mit
R = C₁-C₄-Alkyl oder Phenyl, X = Cl, Br, OCH₃, OC₂H₅, n und m = 0 bis 4
modifiziert. Besonders bevorzugt ist das Kieselgel mit Dimethylchlorsilan und/oder Methyltrichlorsilan modifiziert.

Die Sorptionsvorrichtung enthält bevorzugt einen Fest- oder Wanderbettadsorber. Ebenso ist es möglich, dass die Sorptionseinheit einen Wirbelschichtadsorber aufweist.

Das Kieselgel kann vorzugsweise weitere oxidische Zusätze, wie z.B. TiO₂, Al₂O₃, FeO oder CeO₂ enthalten.

Die Erfindung umfasst ebenso die Verwendung von hydrophobiertem Kieselgel als selektiven Sorbens für siliziumorganische Verbindungen in biogenen und/oder anthropogenen Gasen. Hierzu zählen insbesondere biogene und anthropogene, methanhaltige Gase, wie z.B. Biogas, Klärgas oder Deponiegas.

Anhand des nachfolgenden Beispiels soll der erfindungsgemäße Lösungsweg detailliert erläutert werden, ohne diesen auf die hier beschriebene Variante einzuschränken.

### Beispiel

### 1. Herstellung des hydrophobierten Kieselgels

Die exotherme Silanisierung des Kieselgels erfolgt im Batch-Verfahren. Geeignete, marktgängige Fällungskieselgele mit einer definierter Porenradien- und Korngrößenverteilung werden definiert getrocknet und wieder befeuchtet.

Anschließend erfolgt die Oberflächensilanisierung des Kieselgels mittels einer Behandlung mit einer definierten Mischung aus Methylchlorsilanen (technische Qualität, z.B. Dimethyldichlor- und Methyltrichlorsilan) und einem Solvent (beispielsweise Tetrahydrofuran, Diethylether oder Toluol). Der Silanisierungsgrad der Kieselgeloberfläche ist von der Rezeptur der Silanisierungslösung abhängig. Das Solvent wird im geschlossenen Kreislauf gefahren und recycelt. Einzig auszutragendes Abfallprodukt bei der Kieselgelsilanisierung ist HCl, das ausgeschleust und neutralisiert wird. Abschließend erfolgt eine Trocknung des Kieselgels.

Alternativ kann die Herstellung auch lösungsmittelfrei durch Gasphasen-Reaktion mit dem Kieselgel, mit und ohne Trägergas durchgeführt werden. Auch hier wird unumgesetztes Silan durch Kondensation rückgewonnen und rückgeführt, sowie das anfallende Koppelprodukt i.a. HCI ausgeschleust und neutralisiert.

### 2. Anwendung des hydrophobierten Kieselgels

Das hydrophobierte Kieselgel wird wie die bisher verwendete Aktivkohle in Festbettadsorbern eingesetzt. Dazu ist bei bereits installierten Anlagen lediglich das alte Sorbensmaterial (Aktivkohle) durch hydrophobiertes Kieselgel zu ersetzen.

Die in biogenen bzw. anthropogenen methanhaltigen Gasen enthaltenen organischen Siliziumverbindungen adsorbieren an der Oberfläche des Kieselgels und werden aus dem Gas entfernt. Aromaten und Wasserdampf werden dabei nicht in nennenswertem Maße mit adsorbiert.

### 3. Verfahren zur Regenerierung des hydrophobierten Kieselgels

Die extraktive Regenerierung von benutztem silanisierten Kieselgel erfolgt mit geeigneten Eluierungsmitteln im Batch-Verfahren, vorzugsweise in einer Eluierungsapparatur nach Soxhlet.

## Patentansprüche

1. Verwendung von hydrophobiertem Kieselgel als Sorbens für die selektive Entfernung siliziumorganischer Verbindungen aus biogenen und/oder anthropogenen Gasen.

2. Verwendung nach Anspruch 1,
**dadurch gekennzeichnet, dass** das Kieselgel eine Korngröße im Bereich von 0,2 bis 5 mm aufweist.

3. Verwendung nach einem der Ansprüche 1 oder 2,
**dadurch gekennzeichnet, dass** das Kieselgel einen mittleren Porenradius von 1 bis 40 nm besitzt.

4. Verwendung nach einem der Ansprüche 1 bis 3,
**dadurch gekennzeichnet, dass** das Kieselgel sila nisiert ist.

5. Verwendung nach einem der Ansprüche 1 bis 4,
**dadurch gekennzeichnet, dass** das Kieselgel mit Silanverbindungen der allgemeinen Formel I
RₙSiXₘH₄₋ₙ₋ₘ I
mit
R = C₁-C₄-Alkyl oder Phenyl, X = Cl, Br, OCH₃, OC₂H₅, n und m = 0 bis 4
modifiziert ist.

6. Verwendung nach einem der Ansprüche 1 bis 5,
**dadurch gekennzeichnet, dass** das Kieselgel mit Dimethylchlorsilan und/oder Methyltrichlorsilan modifiziert ist.

7. Verwendung nach einem der Ansprüche 1 bis 6,
**dadurch gekennzeichnet, dass** das Kieselgel wei tere oxidische Zusätze enthält.

8. Verwendung nach einem der Ansprüche 1 bis 7,
**dadurch gekennzeichnet, dass** das Sorbens regene rierbar ist.

9. Verwendung nach einem der Ansprüche 1 bis 8,
**dadurch gekennzeichnet, dass** die biogenen und/oder anthropogenen Gase methanhaltig sind.

10. Verwendung nach einem der Ansprüche 1 bis 9,
**dadurch gekennzeichnet, dass** das Sorbens in einem Fest- oder Wanderbettadsorber oder einem Wirbelschichtadsorber verwendet wird.

## Claims

1. Use of hydrophobised silica gel as a sorbent for the selective removal of organic silicon compounds from biogenic and/or anthropogenic gases.

2. Use according to claim 1, **characterised in that** the silica gel has a particle size in the range from 0.2 to 5 mm.

3. Use according to either claim 1 or claim 2, **characterised in that** the silica gel has an average pore radius from 1 to 40 nm.

4. Use according to any one of claims 1 to 3, **characterised in that** the silica gel is silanised.

5. Use according to any one of claims 1 to 4, **characterised in that** the silica gel is modified with silane compounds of the general formula I
RₙSiXₘH₄₋ₙ₋ₘ I
where
R = C₁-C₄ alkyl or phenyl, X = Cl, Br, OCH₃, OC₂H₅, n and m = 0 to 4.

6. Use according to any one of claims 1 to 5, **characterised in that** the silica gel is modified with dimethylchlorosilane and/or methyltrichlorosilane.

7. Use according to any one of claims 1 to 6, **characterised in that** the silica gel contains further oxide additions.

8. Use according to any one of claims 1 to 7, **characterised in that** the sorbent is regenerable.

9. Use according to any one of claims 1 to 8, **characterised in that** the biogenic and/or anthropogenic gases contain methane.

10. Use according to any one of claims 1 to 9, **characterised in that** the sorbent is used in a fixed-bed or moving-bed adsorber or in a fluidised-bed adsorber.

## Revendications

1. Utilisation d'un gel de silice hydrophobisé comme sorbant pour éliminer sélectivement des composés organosilicés de gaz biogènes et/ou anthropogènes.

2. Utilisation selon la revendication 1,
**caractérisée en ce que** le gel de silice présente une granulométrie dans la plage de 0,2 à 5 mm.

3. Utilisation selon l'une quelconque des revendications 1 ou 2,
**caractérisée en ce que** le gel de silice présente un rayon de pores moyen de 1 à 40 nm.

4. Utilisation selon l'une quelconque des revendications 1 à 3,
**caractérisée en ce que** le gel de silice est silanisé.

5. Utilisation selon l'une quelconque des revendications 1 à 4,
**caractérisée en ce que** le gel de silice est modifié par des composés silane de formule générale I :
RₙSiXₘH₄₋ₙ₋ₘ I
avec
R = alkyle en C₁-C₄ ou phényle, X = Cl, Br, OCH₃, OC₂H₅, n et m = 0 à 4

6. Utilisation selon l'une quelconque des revendications 1 à 5,
**caractérisée en ce que** le gel de silice est modifié par du diméthylchlorosilane et/ou du méthyltrichlorosilane.

7. Utilisation selon l'une quelconque des revendications 1 à 6,
**caractérisée en ce que** le gel de silice contient d'autres additifs oxydés.

8. Utilisation selon l'une quelconque des revendications 1 à 7,
**caractérisée en ce que** le sorbant est régénérable.

9. Utilisation selon l'une quelconque des revendications 1 à 8,
**caractérisée en ce que** les gaz biogènes et/ou anthropogènes contiennent du méthane.

10. Utilisation selon l'une quelconque des revendications 1 à 9,
**caractérisée en ce que** le sorbant est utilisé incorporé à un à lit fixe ou à lit mobile d'adsorbant ou à un à lit fluidisé d'absorbant.
